# EUROPEAN PATENT SPECIFICATION

(11) **EP 4 326 354 B1**
(45) Date of publication and mention of the grant of the patent: **02.07.2025**
(21) Application number: 22722844.2
(22) Date of filing: 19.04.2022
(51) Int. Cl.: A61L 27/24, A61L 27/56, A61L 27/52, A61L 27/38, A61L 31/04, A61L 31/14, A23L 13/00

(54) **COLLAGEN HYDROGELS USEFUL AS CELL CARRIERS**
KOLLAGENHYDROGELE ZUR VERWENDUNG ALS ZELLTRÄGER
HYDROGELS DE COLLAGÈNE UTILES EN TANT QUE SUPPORTS DE CELLULES

(30) Priority: 19.04.2021 EP 21382331
(43) Date of publication of application: 28.02.2024
(73) Proprietor: Viscofan, S.A., 31192 Tajonar (Navarra) (ES)
(72) Inventor: GUEMBE LAPUENTE, Amaia, 31192 TAJONAR (ES); ZÚÑIGA ARRARÁS, Teresa, 69469 WEINHEIM (DE); RECALDE IRURZUN, José Ignacio, 31192 TAJONAR (Aranguren) (ES); IZCO ZARATIEGUI, Jesús Maria, 31192 TAJONAR (ES)
(74) Representative: ZBM Patents - Zea, Barlocci & Markvardsen
(86) International application number: PCT/EP2022/060241
(87) International publication number: WO 2022/223518

(56) References cited:
- WO-A1-2012/030202
- WO-A1-2022/069772
- US-B1- 6 706 684
- JAFARI HAFEZ ET AL: "Fish Collagen: Extraction, Characterization, and Applications for Biomaterials Engineering", POLYMERS, vol. 12, no. 10, 28 September 2020 (2020-09-28), pages 2230, XP055846678, DOI: 10.3390/polym12102230

## Description

This application claims the benefit of European Patent Application EP21382331.3 filed on 19 April 2021.

### Technical Field

The present invention relates to a collagen hydrogel, a process for its preparation, as well as its use as scaffold for the cultivation of cells.

### Background Art

Carriers for the cultivation of biological cells are generally known in the art. Such carriers are often referred to as matrix or scaffold. These carriers provide the breeding ground or, in general, the basis on which the cells grow in cell culture.

Collagen-containing compounds are known type of cell carriers. Collagen consists of amino acids bound together to form a triple helix of elongated fibril known as a collagen helix. Collagen is the most abundant protein in the mammalian body making up from 25 to 35% of the whole-body protein content. It is mostly found in connective tissue such as cartilage, bones, tendons, ligaments, and skin. Depending upon the degree of mineralization, collagen tissues may be rigid (bone), compliant (tendon), or have a gradient from rigid to compliant (cartilage). Collagen is also abundant in corneas, blood vessels, the gut, intervertebral discs, and the dentin in teeth. In muscle tissue, it serves as a major component of the endomysium. Collagen constitutes one to two percent of muscle tissue and accounts for 6% of the weight of strong, tendinous, muscles. The fibroblast is the most common cell that creates collagen. Collagen represents the main structural protein of the extracellular matrix, a protein scaffold conveying structure and stability to tissues, offering sites for cells to attach to and influencing and supporting the adherent cells.

Collagen-based biomaterials from animal sources have already been used in medicine for several years now. Especially in the clinically applicable products for hemostasis or in different areas of plastic surgery, collagens materials have been proposed as a carrier material. However, the collagen-only compositions are based on soluble collagen and are often not sufficiently consistent and firm, i.e., they lack mechanical strength without the aid of crosslinking agents or do not have a native configuration, i.e., gelatin.

In conventional production, collagen is extracted in a solubilized form by acids or enzyme treatment. The resulting subunits, the triple helical tropocollagens, must be re-organized *in vitro* yielding only fibrils and thin fibers that merely qualify to build up hydrogels or thin coatings.

A hydrogel is a water-containing but water-insoluble polymer, whose molecules are chemically (e.g., through covalent or ionic bonds) or physically (e.g., by means interlinking the polymer chains) linked to produce a three-dimensional network. Due to integral hydrophilic polymer components, they expand in water under a considerable intake of volume but without losing their material cohesion. However, the disadvantage of hydrogels is that, given their enormous water-retaining capacity, they are mechanically unstable. Collagen hydrogels have been proposed as carrier for the cultivation of cells. However, due to the lack of mechanical stability and the natural structure of the complex fibers, and due to the fact that the hydrogels often condense during the colonization with cells, they are very limited in their use.

Collagen properties may be modified in several ways resulting in the formation of ionic bonds, hydrogen bonds or covalent bonds. The creation of reticulation increases the biodegradation time of the collagenic material and the mechanical strength of the collagen fibers, while reducing the water absorptivity, the solubility, and the rate of enzymatic degradation of these fibers. The collagen may be crosslinked either by physical methods or by chemical methods.

The chemical methods use crosslinking agents such as aldehyde compounds, carbodiimides, diisocyanates, or azide derivatives. They have the drawback that some of those agents are highly toxic and the extra crosslinks compromise the natural structure of the collagen fibers and are, therefore, antagonistic to their biocompatibility and biodegradability.

Among the physical methods, beta or gamma irradiation has been used, not only to sterilize but to reticulate the collagen materials. When beta radiation is used to sterilize dehydrated collagen, it leads to a material which resistance is difficult to predict. US6706684B1 describes a process for the preparation of a collagen material whose speed of biodegradation *in vivo* and mechanical properties are modulable based on irradiating a collagen material in wet state with beta radiation. The collagen material used may be native solubilized collagen at acid pH, or as obtained after treatment by digestion with pepsin. It can be bovine collagen type I or III or mixtures thereof, collagen modified by breakage through oxidation, mainly by means of periodic acid; collagen that has partially lost its helical structure through heating, functionalized collagen, or collagen with a macromolecular hydrophilic additive. In all these cases soluble collagen is used. All these collagen materials are disclosed as useful for the prevention of post-operative adhesion and/or the healing of wounds and are prepared from soluble collagen.

Another way disclosed in the art to increase the mechanical properties of the collagen materials is to assembly the collagen to other materials. US6706684B1 previously mentioned discloses an assembly in which the collagenic component intended to be structured by irradiation has been combined beforehand with a network of undenatured collagen fibers, which is in the form of a compacted compress. Other alternatives are based on the formation of scaffold with other components. For instance, P.N. Oliveira et al., disclose a porous collagen/chitosan scaffolds with different Collagen : Chitosan (Coll:Ch) ratios, which were prepared by freeze drying followed by self-crosslinking via dehydrothermal treatment (DHT) and subsequent sterilization with beta radiation. It also discloses that the collagen material obtained is used as biomaterials for tissue engineering (cf. P.N. Oliveira et al; "Self-crosslinked fibrous collagen/chitosan blends: Processing, properties evaluation and monitoring of degradation by bi-fluorescence imaging"; International Journal of Biological Macromolecules, 2019, pp. 353-367).

Despite what has already been disclosed in the art, there is still the need of providing collagen materials that can be produced economically on a large scale without undesirable toxicities, with high quality and stability, and which properties that can be modulated to adapt the collagen material to the intended use.

### Summary of Invention

The present inventors have developed non-soluble and native fibrous collagen hydrogel structures that can be prepared without the addition of stabilizing chemicals and yielding to resistant structures without the need to use crosslinkers, which further show a wide range of mechanical strength. These hydrogels are firm 3D structures with a series of micropores that show good stability, in particular, good integrity in a culture medium. They may interact with other biomolecules, and mediates cell adhesion and, therefore, are useful for cell support.

They can be prepared from a dispersion in acidic medium of native fibrillar collagen of a certain size, in particular, native Type I collagen fibers dispersed in water in a concentration comprised between 1-15% in weight, where the fibers have a greater size than in the case of soluble collagen. The acidic mass comprises fibers, wherein at least 90% of the mass of the fibers have a length comprised in a range from 1 µm to 2500 µm, and a fiber diameter comprised in a range from 0.1 to 150 µm, measured at a pH between 1 and 2. More precisely, the acidic mass comprises fibers, wherein at least 90% of the total mass of the fibers have a length comprised in a range from 10 µm to 2500 µm and a fiber diameter comprised in a range from 5 to 80 µm measured at a pH between 1 and 2; and wherein the median of the fiber length in the total mass of fibers of collagen is comprised between 100 and 900 µm.

Unlike the collagen generally used in the prior art, which is generally in the form of gelatin, (collagen that has been irreversibly hydrolyzed) or is enzymatically treated, the collagen type I used in the present invention as starting material is characterized by being highly native and by the size of the fibers. This native fibrillar collagen starting material used in the present invention is generally used in the food industry, for example, in the area of ham production as a separating film between net and meat.

Thus, unlike US6706684B1 which uses soluble collagen (the one commonly used in the prior art for related purposes), the collagen used in the present invention as starting material to prepare the hydrogel is a non-soluble collagen, which is more economical since it comes from the food industry. It is unexpected that such non-soluble collagen starting material could yield after an appropriate treatment to collagen hydrogels such as those of the invention which are particularly suitable for the cultivation of biological material. The use of non-soluble collagen contributes to provide to the hydrogel the appropriate mechanical properties to be used as cell support. The treatment carried out to such collagen (neutralization of the previous acidic mass, adjustment to physiological pH, and application of beta radiation) contributes to control the stiffness of the final hydrogel probably through the degree of cross-linking obtained.

Accordingly, a first aspect of the present invention relates to a collagen hydrogel which comprises a 3D fibrillar structure with micropores, obtainable by a process comprising the following steps: a) providing an acidic mass of native Type I collagen fibers dispersed in water in a concentration comprised between 1-15% in weight; b) neutralizing the acidic mass to adjust the pH to physiological pH; and c) applying beta radiation to the neutralized mass of step b) in an amount of up to 50 KGy (i.e. equal to or lower than 50KGy); wherein the acidic mass has a pH comprised between 0.5-5 and comprises fibers, wherein at least 90% of the total mass of the fibers have a length comprised in a range from 10 µm to 2500 µm and a fiber diameter comprised in a range from 5 to 80 µm, measured at a pH between 1 and 2, and wherein the median of the fiber length in the total mass of fibers of collagen is comprised between 100 and 900 µm. The size of the fibers has been measured with a Sympatec QICPIC/Lixell particle analyzer as disclosed in Example 3A.

A second aspect of the present invention relates to a process for preparing a collagen hydrogel as defined above, which comprises the following steps: a) providing an acidic mass of native Type I collagen fibers dispersed in water as defined above, b) neutralizing the acidic mass to adjust the pH to physiological pH; and c) applying beta radiation to the neutralized mass of step b) in an amount of up to 50 KGy. The preparation of the collagen hydrogel according to the invention can be reproduced on a large scale and is of a constant high quality.

The collagen hydrogel of the present invention stands out due to its good biocompatibility, and its high mechanical stability, resulting in a wide range of applications. In particular, due to its central role in cell adhesion and organ formation, it is an ideal substrate for cell culture and tissue engineering. Thus, a third aspect of the present invention relates to the use of the collagen hydrogel as defined above as support for cell growth or as scaffold for cultured meat.

A fourth aspect of the present invention relates to a method for the cultivation of biological material, comprising the following steps: a) providing a carrier material which is a collagen hydrogel as defined above; b) contacting said biological material with the collagen hydrogel of step a); and c) incubating said biological material with the collagen hydrogel material under cultivation conditions.

A fifth aspect of the present invention relates to a collagen hydrogel as defined above comprising adhered cell layers for use in biomedical applications such as cell based therapies or as a combined advanced therapy medicinal products (ATMP).

### Brief Description of Drawings

FIG. 1 shows two photographs (images a) and b)) of the hydrogels of the present invention FIG. 1a) from collagen hydrogel from a 4% mass. FIG 1 b) from a 2% mass.
FIG. 2 images 1-9 were obtained by optical microscopy and illustrate the size of the fibers of the hydrogels of the present invention (images 1, 2, 5, 6), and the comparative with soluble collagen (images 3, 4 and 7) and hydrogel solubilized in acid ( images 8 and 9). Cont. in the figures means continuation.
FIG.3 images 1-4 shows photographs of the internal structure of hydrogels of the present invention at 1, 2, 3 and 4% collagen by ESEM technology.
FIG. 4 shows the rheology results for a hydrogel according to the invention.
FIG. 5 has two Images, 1 and 2, which show the cell culture on the collagen gels after DiIC12 staining with the I929 line, obtained 2 and 5 days after seeding, respectively.
FIG. 6 is a graphical representation of cell proliferation on the different gels at days 1, 6 and 10, respectively.
FIG.7 shows a 293T cell line on 4% hydrogels previously irradiated at 25 KGy.

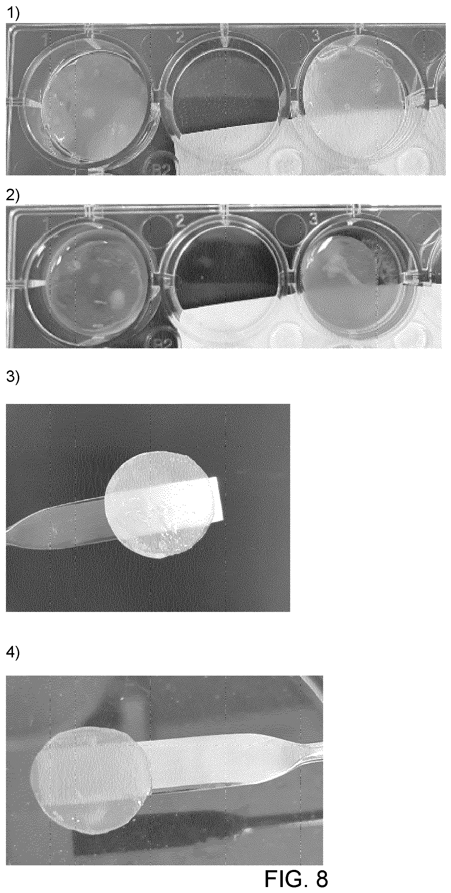

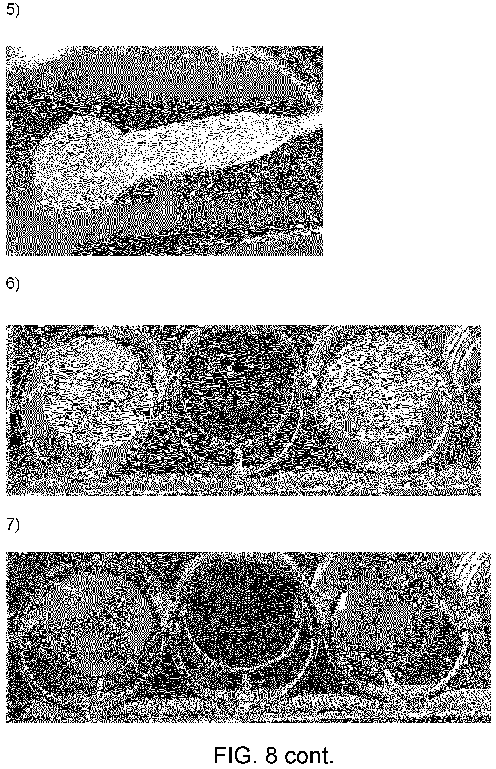

FIG. 8 Images 1-7 illustrates the stability of the hydrogels of the invention in a culture medium. Image 1: Hydrogels before immersion in culture medium. Left: 2% collagen irradiated at 10kGy. Right: 2% collagen irradiated at 25kGy. Image 2: Hydrogels incubated for 7 days in culture medium. Left: 2% collagen irradiated at 10kGy. Right: 2% of collagen irradiated at 25kGy. Image 3: 2% collagen hydrogels irradiated at 10kGy before immersion in culture medium. Image 4: 2% collagen hydrogels irradiated at 10kGy incubated for 7 days in culture medium. Image 5: 2% collagen hydrogels irradiated at 25kGy incubated 7 days in culture medium. Image 6: Hydrogels before immersion in culture medium. Left: 4% collagen irradiated at 10kGy. Right: 4% collagen irradiated at 25kGy.Image 7: Hydrogels incubated for 7 days in culture medium. Left: 4% collagen irradiated at 10kGy. Right: 4% collagen irradiated at 25kGy.

### Detailed description of the invention

### Definitions:

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" or "approximately" as used herein refers to a range of values ± 10% of a specified value. For example, the expression "about 10" or "around 10" includes ± 10% of 10, i.e., from 9 to 11.

The term "comprised between" or "comprised in a range from x to y" as used herein refers to a range of values including the end points of the range. Any range of values disclosed herein regardless of the wording used, are encompassing the end points of the range in its scope.

The word "comprise" for the purposes of the present invention encompasses the case of "consisting of".

The term "at least" in the expression "at least x% in weight of the fibers" means a percentage equal to or higher than the given value.

The collagen molecule is an elongated molecule made up of three polypeptide chains coiled together constituting a triple helix stabilized by hydrogen bridge interactions or bonds, this is the structure of native collagen. When collagen is denatured, for example by heating it in the presence of water or by heating it to extreme pH values, these chains separate and dissolve, forming a gelatin. The formation of gelatin is due to the breaking of the hydrogen bonds that stabilize the collagen triple helix, the process of transformation of collagen into gelatin is considered a typical denaturation process. In the present dispersion of collagen fibers there is no gelatin, or its presence is not significant.

In the present description "native collagen" is understood to mean collagen which has not been denatured, hydrolyzed, or gelatinized, totally or significantly. The collagen fiber of the hydrogel of the present invention maintains the triple helix molecular structure of the units of collagen without denaturing.

The term "native Type I collagen" is understood as being collagen which is in high extent type I collagen, it may contain small amounts of other collagens such as for example collagen III. Generally, the amount of collagen I is equal to or higher than 90% in weight, preferably, equal to or higher than 92% in weight, more preferably, equal to or higher than 95% in weight, even more preferably, equal to or higher than 98% in weight, and even still more preferably, equal to or higher than 99% in weight.

The term "physiological pH" is understood as the pH that normally prevails in the human body. It is approximately 7.3-7.4.

The pH measurements of the process of the present invention have been carried out with a pH-meter (sample in NaCl 0.5% measured at 20 °C).

The term "micropore" is understood as pores with pore diameters in the micrometric range. Generally, in the hydrogels of the present invention the pores have a pore diameter equal to or greater than 50 µm. Porosity is seen by environmental scanning electron microscopy (ESEM) technology.

The term "dispersed" in the sentence "acidic mass of native Type I collagen fibers dispersed in water" means a state that the mass contains collagen fibers not dissolved but dispersed in pH concentration conditions.

Unless otherwise stated, all percentages mentioned herein are expressed in weight with respect to the total weight of the collagen acidic mass or hydrogel, provided that the sum of the amounts of the components is equal to 100%.

It is part of the invention a collagen hydrogel which comprises a 3D fibrillar structure with micropores obtainable by a process comprising: a) providing an acidic mass of native Type I collagen fibers dispersed in water in a concentration comprised between 1-15% in weight; b) neutralizing the acidic mass to adjust the pH to physiological pH; and c) applying beta radiation to the neutralized mass of step b) in an amount of up to 50 KGy; wherein: the acidic mass has a pH comprised between 0.5-5; and comprises fibers, wherein at least 90% of the mass of the fibers have a length comprised in a range from 10 µm to 2500 µm and a fiber diameter comprised in a range from 5 to 80 µm measured at a pH between 1 and 2, and wherein the median of the fiber length in the total mass of fibers of collagen is comprised between 100 and 900 µm. Generally, the process is carried out at room temperature (20-30 °C, generally 20-25 °C).

This acidic mass of insoluble collagen type I is also characterized in that the fibers are in highly preserved state, long, strong, and native.

In a particular embodiment in combination with any of the embodiments above or below, at least 90% of the mass of the collagen fibers which are dispersed in the acidic mass have a fiber length which is comprised in a range from 20 µm to 2500 µm. In another particular embodiment in combination with any of the embodiments above or below, at least 90% of the mass of the collagen fibers dispersed in the acidic mass have a fiber length which is comprised in a range from 50 µm to 2500 µm.

In another particular embodiment, optionally in combination with any of the embodiments above or below, at least 80% of the mass of the collagen fibers dispersed in the acidic mass have a fiber length comprised in a range from 50 µm to 1500 µm.

In another particular embodiment in combination with any of the embodiments above or below, at least 80% of the mass of the collagen fibers dispersed in the acidic mass have a fiber length comprised in a range from 50 µm to 1300 µm. In another particular embodiment in combination with any of the embodiments above or below, at least 80% of the mass of the collagen fibers dispersed in the acidic mass have a fiber length comprised in a range from 50 µm to 1250 µm. In a particular embodiment in combination with any of the embodiments above or below, at least 80 % of the mass of the collagen fibers dispersed in water have a fiber length ranging from 90 to 1235 µm. In another particular embodiment, 80 % of the collagen fibers dispersed in water have a fiber length ranging from 99 to 1235 µm.

In another particular embodiment in combination with any of the embodiments above or below, the length below which 90% of the fibers lies is comprised in a range from 1500 to 2500 µm.

The median of the fiber length in the total mass of fibers of collagen is comprised in range from 100 to 900 µm, i.e., it is a value from 100 to 900 µm. This means that 50% of the mass of the fibers of collagen is due to fibers shorter than the median value and 50% of the mass of the fibers of collagen is due to fibers longer than the median value. These percentages refer to the percentages of the cumulative distribution of collagen fibers in the total acidic mass (length distribution).In another particular embodiment in combination with any of the embodiments above or below, the median of the fiber length in the total mass of fibers of collagen is comprised in range from 100 to 500 µm. In another particular embodiment in combination with any of the embodiments above or below, the median of the fiber length in the total mass of fibers of collagen , is comprised between 200 and 400 µm.

Furthermore, at least 90% of the mass of the collagen fibers which are dispersed in the acidic mass have a fiber diameter which is comprised in a range from 5 to 80 µm. In a particular embodiment, optionally in combination with any of the embodiments above or below, at least 80% of the mass of the collagen fibers dispersed in the acidic mass have a fiber diameter comprised in a range from 5 µm to 50µm.

In a particular embodiment in combination with any of the embodiments above or below, at least 80% of the mass of the collagen fibers dispersed in the acidic mass have a diameter comprised in a range from 5 to 35 µm. In another particular embodiment, at least 80% of the mass of the collagen fibers dispersed in the acidic mass have a diameter comprised in a range from 10 to 31 µm. In another particular embodiment, 80% of the mass of the collagen fibers dispersed in the acidic mass have a diameter comprised in a range from 10 to 31 µm.

In another particular embodiment in combination with any of the embodiments above or below, the diameter below which 90% of the fibers lies, is comprised in a range from 80 to 150µm. In another particular embodiment in combination with any of the embodiments above or below, the median of the fiber diameter in the total mass of fibers of collagen is comprised in range from 10 to 25 µm, i.e., it is a value from 10 to 25 µm. This means that 50% of the mass of the fibers of collagen is due to fibers thinner than the median value and 50% of the mass of the fibers of collagen is due to fibers thicker than the median value. These percentages refer to the percentages of the cumulative distribution of collagen fibers in the total acidic mass (diameter distribution). In another particular embodiment in combination with any of the embodiments above or below, the median value of the fiber diameter in the total mass of fibers of collagen , is comprised between 15 and 25 µm.

In a particular embodiment in combination with any of the embodiments above or below, the acidic mass has a concentration comprised between 1-10 % in weight of native Type I collagen. In another particular embodiment, the acidic mass has a concentration comprised between 1-8% in weight of native Type I collagen. In another particular embodiment, the acidic mass has a concentration comprised between 1-5% in weight of native Type I collagen. In another particular embodiment, the acidic mass has a concentration comprised between 2-4% in weight of native Type I collagen. The acidic mass may be in any specific concentration comprised in the interval between 1-15% in weight, i.e., 1%, 2%, 3%, 4%, 5%, 6%,7%, 8%, 9%, 10%, 11;%, 12%, 13%, 14% and 15%.

In a particular embodiment in combination with any of the embodiments above or below, the acidic mass has a pH comprised between 1 and 3. The neutralization is carried out by adding a base until the required pH with an appropriate alkaline agent such as sodium hydroxide, ammonium chloride, potassium and sodium phosphate buffers, tris buffer, calcium carbonate, or 4-(2-hydroxyethyl)-1-piperazineethanesulfonic acid (Hepes), etc.

The dose of beta radiation is applied in a range equal to or below than 50 KGy. In a particular embodiment in combination with any of the embodiments above or below, the beta radiation applied is in the range between 1-50 KGy. In another particular embodiment in combination with any of the embodiments above or below, the beta radiation applied is in the range between 5-50Kgy. In another particular embodiment in combination with any of the embodiments above or below, the beta radiation applied is in the range between 10-50Kgy. In another particular embodiment in combination with any of the embodiments above or below, the beta radiation applied is in the range between 10-25 KGy. In another particular embodiment in combination with any of the embodiments above or below, the beta radiation applied is in the range between 15-25 KGy.

In a particular embodiment, the beta radiation in step c) is applied to collagen in wet state. In a particular embodiment, the beta radiation is applied directly to the collagen mass of step b).

In a particular embodiment in combination with any of the embodiments above or below, the collagen hydrogel according to the invention is absent of chemical crosslinking.

It is also part of the present invention, a process for preparing a collagen hydrogel as defined above, which comprises the following steps: a) providing an acidic mass of native Type I collagen fibers dispersed in water as defined above, b) neutralizing the acidic mass adjusting the pH to physiological pH; and c) applying beta radiation to the neutralized mass of step b) in a range of up to 50 KGy. Generally, the process is carried out at room temperature (20-25 °C).

In a particular embodiment of the process in combination with any of the embodiments above or below, the beta radiation is in a range comprised between 5 and 50 KGy.

In a particular embodiment of the process in combination with any of the embodiments above or below, the beta radiation is applied directly to the collagen mass of step b), without carrying out a lyophilization step.

The acidic mass used as starting material is obtained from a biological source of collagen, usually consisting of large bundles and/or fibrillar aggregates subjected to treatments which, preserving the native structure, aimed at obtaining fibers of smaller diameter and length. This collagen is insoluble in water.

Accordingly, the acidic mass used in the process of the present invention can be previously prepared by a process which comprises the following steps: a) washing and cutting up a tissue containing collagen; b) chemically macerating the chopped tissue in a controlled manner; c) washing the product obtained from step b) with water; d) adjusting the pH of the washed product obtained in c) to values between 0.5 and 5 to swell the washed product of step c); e) mechanically mincing the product of step d); and f) dispersing in water to a desired concentration, in particular between 1% and 15% by weight.

In a particular embodiment, the collagen-containing tissue is connective tissue. Generally, the connective tissue is selected from: dermis, bone, tendon, cartilage, and intestine. In a particular embodiment, the connective tissue is dermis tissue and more preferably the collagen is extracted from the layer called corium. In a particular embodiment, the connective tissue is of bovine, ovine, porcine, avian, fish origin or a mixture thereof. In another particular embodiment, the connective tissue is of bovine origin and from an animal of an age of more than one year, generally, between 1 and 3 years.

Generally, before step a) when the connective tissue is the dermis, the dermis is depilated and bleached. An example of an oxidizing agent for the bleaching step is dilute hydrogen peroxide.

Step b) can be performed in the presence of an alkaline agent e.g., calcium hydroxide or sodium hydroxide, in the presence or absence of enzymes. In a particular embodiment, step b) is performed with sulfide salts and alkaline treatments, such as for example Na₂S and Ca(OH)₂.

In a particular embodiment, the acids used to acidify in step d) are selected from: hydrochloric, lactic, acetic, and citric. In another particular embodiment, it is acidified to pH between 1 and 5.

A commercial collagen mass such as Viscolma^{®} (up to 15% collagen fiber content in water) can also be used as a collagen acidic mass.

The specific particular embodiments of the product defined by its process are also particular embodiments of the process for its obtaining.

The structure of the collagen hydrogels obtainable by the process of the present invention is shown in the examples. Thus, for instance, Example 4A illustrates that the collagen hydrogel of the present invention comprises fibers in its structure, in this specific case of the example having an average length around 600 µm and an average diameter around 15 µm.

In order to use the hydrogels in biomedical applications, in particular as a support for cell growth, a minimum consistency and integrity of the hydrogel has to be ensured in order to maintain the firmness needed for the cells to grow. This requires a minimum elastic modulus. The elastic modulus or storage modulus (G') is associated with the energy stored in the material. The viscous or loss modulus (G") is associated with the energy dissipated by the material.

All tissues have distinct intrinsic physical properties, which are important in their structure and function. The stiffest tissues of the body are tooth and bone muscle is intermediate, and among the softest are lung and brain. The stiffness of the hydrogels of the present invention can be modulated to have the required stiffness to the intended use. Advantageously, the collagen hydrogels of the present invention can be prepared with a wide range of stiffness.

The examples illustrate a collagen hydrogel according to the invention which have been irradiated with 25 KGy and has an elastic modulus G' ranging from 2500 Pa to 70000Pa in a 1% - 10% in weight collagen content hydrogel, i.e., with a broad spectrum of degrees of stiffness) and a viscous modulus G" ranging from 90 Pa to 4730Pa.

It is also part of the invention the use of the collagen hydrogel as defined above as support for cell growth.

As the inventors have found out, the cultivation of biological cells is particularly successful at the specified pH values. Thus, the collagen hydrogel shows a pH value that lies in the physiological area and, therefore, provides a setting that broadly resembles the natural environment of the biological cells. In this case, it is very advantageous for the collagen to be native, since in this way it more closely resembles the original collagen structure, replicating with greater similarity the original environment in which cell proliferation takes place in the organism. Besides, the hydrogel of the present invention is sufficiently rigid to remain intact and firm over time at physiological pH.

It is also part of the invention a method for the cultivation of biological material, comprising the following steps: a) providing a carrier material which is a collagen hydrogel material as defined above; b) contacting said biological material with the collagen hydrogel material of step a); and c) incubating said biological material with the collagen hydrogel material under cultivation conditions. Suitable cultivation conditions are for instance a temperature of around 37°C and 5% CO₂.

In a particular embodiment the biological material is fibroblasts from murine subcutaneous connective tissue. In another particular embodiment, the biological material is human renal embryonic cells.

The collagen hydrogel of the present invention has good properties in terms of biocompatibility, and handling capabilities. These are important requirements on biomaterials for new therapies in regenerative medicine.

Besides, depending on the application, requirements regarding elasticity, tear-resistance and biodegradability can be quite different. The hydrogel of the present invention made of native fibers is considered appropriate for when cell transfer and implantation is intended,

Thus, it is also part of the invention, a collagen hydrogel as defined above comprising adhered cell layers for biomedical uses such as cell based therapies or as a combined Advanced therapy medicinal products (ATMP).

The collagen hydrogel as defined above comprising adhered cell layers can be for use as implant. The collagen hydrogel to which cells are implanted, can be transplanted immediately or can be transplanted after cultivation.

It is also considered appropriate as scaffold for cultured meat. Thus, this use is also part of the present invention.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Example 1: Preparation of a non-denatured Type I collagen acidic mass

To prepare the collagen mass, 18-26 months old bovine animal skin was subjected to a standard depilation process (e.g., with a sulfur salt (Na2S)). The skin was then split, and the dermis was extracted from which collagen was obtained. The following steps were then followed:
a) a tissue containing the collagen was washed with water, and cut into pieces;
b) the cut tissue was chemically macerated with Na₂S and Ca(OH)₂;
c) the product obtained from step b) was washed with water;
d) the pH of the washed product obtained in c) was adjusted to values between 0.5 and 5, more precisely to a value between 1-3,to swell the washed product of step c):
e) the product of step d) was mechanically minced;
f) and dispersed in water to a concentration by weight, preferably between 1% and 15%.

To find the percentage of collagen in the mass, a known amount of mass was weighed, dried in a vacuum oven at 160°C until a constant weight was obtained, and the resulting dry extract was weighed. The ratio of dry collagen to total mass was calculated.

### Example 2: Preparation of a collagen hydrogel

To prepare the collagen hydrogel, a collagen mass at a collagen concentration of 5 to 10% obtained according to Example 1 was used.

It was diluted with water using a kneader to a concentration of 4% solids, which was calculated according to the method mentioned in Example 1. Subsequently, a 1.5 mm thick film was made and immersed in 50 mM NaOH to coagulate it due to a rise in pH and finally washed with phosphate buffer (PBS) at pH 7.4. The hydrogel was irradiated with beta irradiation at 25 kGy to cross-link the collagen and to sterilize it.

To make the hydrogel from a 2% collagen mass, the same protocol mentioned above was followed, but the mass was diluted to 2% dry collagen.

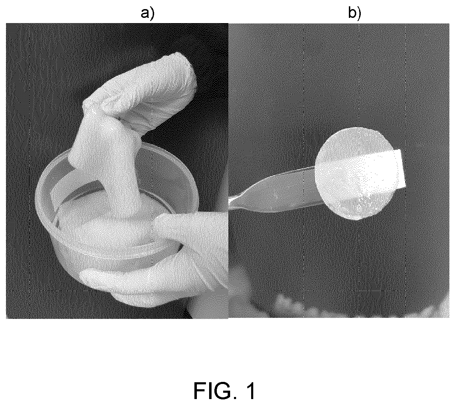

FIG. 1 shows an image of the hydrogels obtained, FIG. 1A shows a hydrogel from a 4% collagen mass and FIG. 1B shows a hydrogel from a 2% collagen mass.

### Example 3: Characterization of the acidic mass of undenatured Type I collagen

### Example 3A: Morphological analysis of the acidic mass of Example 1 by dynamic image analysis. Comparison with soluble collagen and collagen gelatin.

A morphological analysis was performed by dynamic image analysis of the acidic mass of Example 1. For this purpose, it was diluted to 1% in deionized water, and adjusted to a pH of approximately 1.5 with 1 M HCl. The diluted sample was vortexed for 10 min at 2500 rpm and diluted 1/20 with deionized water.

Measurements were performed on a Sympatec QICPIC / Lixell particle analyzer with cuvette (0.5 mm wide) continuous flow in a particle size measurement range of 4 - 2888 µm. Due to the large heterogeneity of particle distribution, typical for collagen suspensions, at least 3 individual measurements of two subsamples were performed. In total, between 1 and 3 million particles were detected.

An evaluation based on fiber length and diameter distribution was performed. The proportion of fibers of a certain length or width in the total mass of collagen fibers in the sample was calculated. Tables 1 and 2 show the length and diameter (in µm) for the 10, 16, 50, 50, 84, and 90% percentages of the cumulative distribution of collagen fibers in the total mass, calculated from the mean values.

**Table 1. Fiber lengths (µm) corresponding to the cumulative percentages of 10, 16, 50, 50, 84 and 90% of total collagen fiber mass.**

| | 10% | 16% | 50% | 84% | 90% |
|---|---|---|---|---|---|
| 531-020 | 99 | 135 | 383 | 989 | 1235 |

**Table 2. Fiber diameters (µm) corresponding to the cumulative percentages of 10, 16, 50, 50, 84 and 90% in weight of total collagen fiber mass.**

| | 10% | 16% | 50% | 84% | 90% |
|---|---|---|---|---|---|
| 531-020 | 10.2 | 12.3 | 19.2 | 27.6 | 30.8 |

The value of the median of the fiber length is 383 µm. This means that half of the fibers contained in the mass have a length greater than 383 µm, with 80% comprising a range from 99 to 1235 µm. And the cumulative distribution of fiber diameters has a median value of 19 µm, with 80% of fibers in a diameter range between 10 µm and 31 µm.

An attempt was made to observe in the optical microscope the soluble collagen solution (comparative Example representing the use of soluble collagen as in US6706684B1) prior to gelation following the manufacturer's instructions and failed to see any collagen fibrils. Therefore, dynamic image analysis was not performed because the fibrils are undetectable.

Gelatin of bovine origin (comparative) was solubilized to perform the same analysis mentioned above and no fiber was detected.

### Example 4: Characterization of the hydrogels of the present invention

### Example 4A: Determination of the fiber size of the hydrogels by optical microscopy.

The size of the fibers of the 2% collagen hydrogel of the present invention made following Example 2, of the gel made from soluble collagen (comparative), and of a 2% gelatin gel (comparative) were determined.

An analysis of the size of the collagen fibers present in the 2% collagen hydrogel was performed. For this purpose, the gel was crushed in deionized water in a 1:10 ratio with the aid of an ultraturrax, a small amount was deposited on a slide and allowed to dry. It was covered with 0.1% Sirius Red (Direct Red 80) dye solution for 1 hour, and then the excess dye was removed. The samples were then observed and quantified under the light microscope.

A 2% bovine gelatin solution (comparative) was prepared by solubilizing the gelatin powder in demineralized water at a temperature of 50°C for a period of 30 minutes. The solution was neutralized to pH 7.4 by addition of 1N NaOH once the powder was dissolved. Then, the same protocol was continued as for the hydrogel, i.e., it was triturated to the same ratio, placed on the slide and stained.

For the measurement of soluble collagen, 0.5% soluble collagen (comparative) was gelled following the manufacturer's protocol to allow the fibers to form, the gel was crumbled and deposited on a slide and the same protocol as above was followed.

**Table 3:**

| | Hydrogel | Gelatin (comparative) | Soluble collagen (comparative) |
|---|---|---|---|
| Average length (µm) | 594 | ND | 174 |
| Minimum length (µm) | 318 | ND | 66,6 |
| Maximum length (µm) | 1214 | ND | 245 |
| Average diameter (µm) | 15,4 | ND | 1,2 |
| Minimum diameter (µm) | 10 | ND | 0,7 |
| Maximum diameter (µm) | 29 | ND | 1,5 |

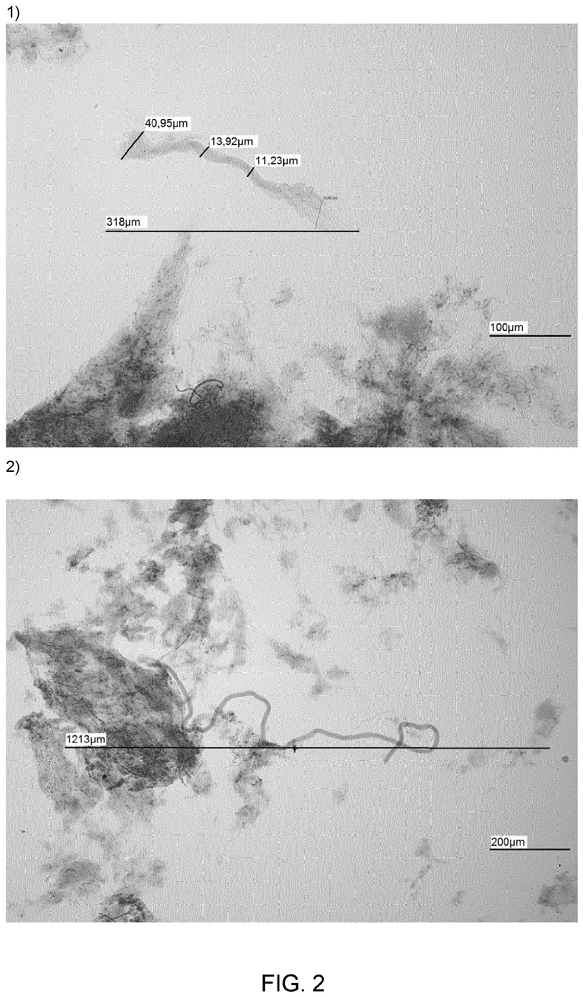

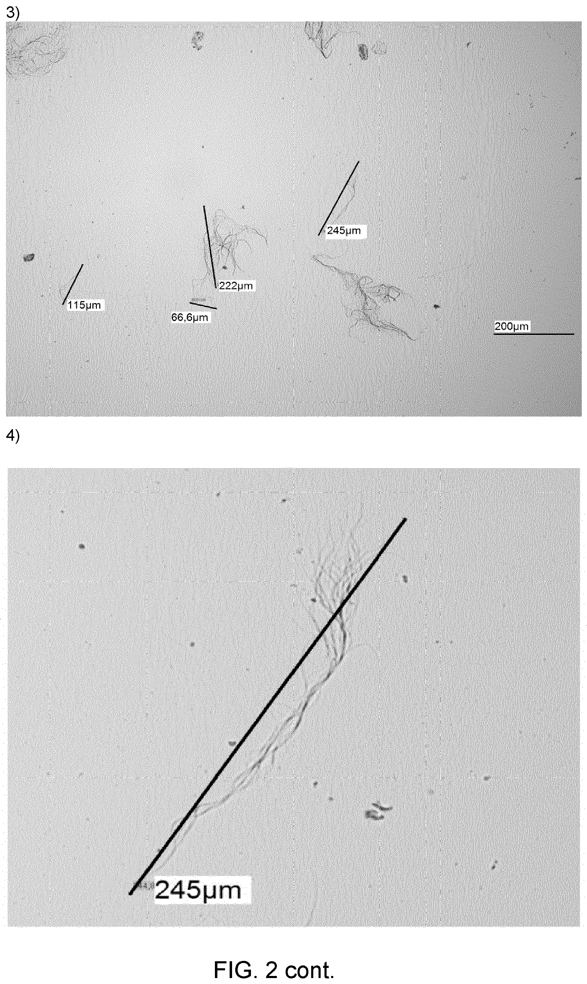

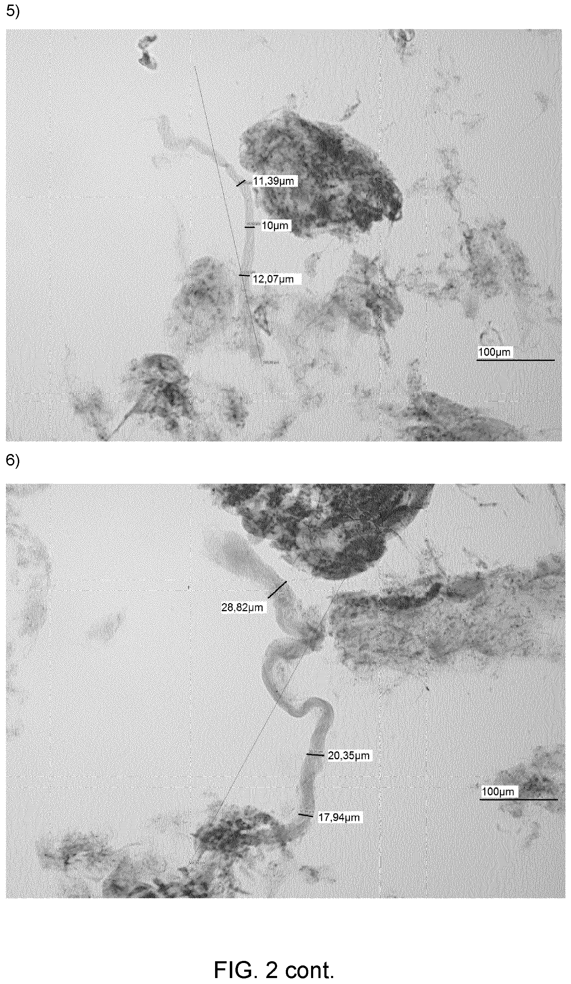

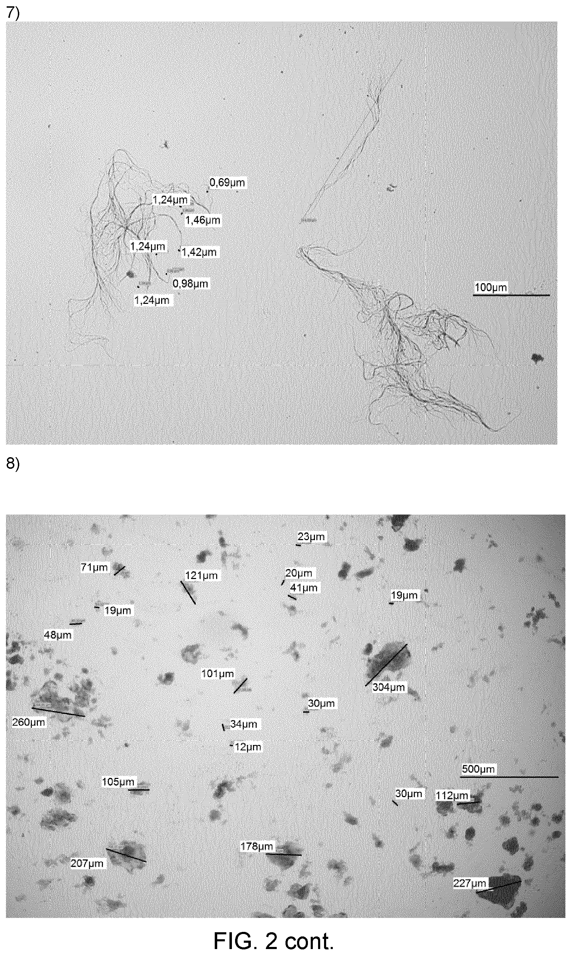

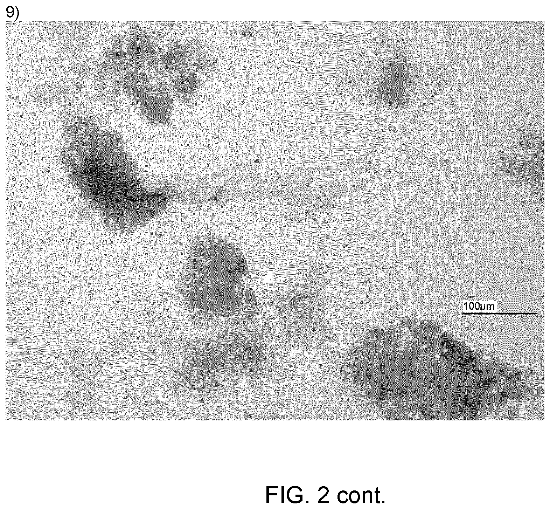

FIG. 2 illustrates the fiber size of the hydrogels of the present invention by optical microscopy. Images 1 and 2 correspond to fiber size of the hydrogel and images 3 and 4 of the soluble collagen (comparative). Images 5 and 6 illustrate the diameter of the hydrogel fibers and image 7 of the soluble collagen.

Thus, fibrils were detected in the hydrogel from 318µm (Image 1) to 1214µm in length (Image 2). In the soluble collagen on the other hand, clusters of fibrils were detected ranging from 67 (Image 3) to 245µm (Image 4).

The diameter of the fibers is clearly differential, with minimum diameters of 10µm (Image 5) and maximum diameters of 29µm (Image 6) in the case of the hydrogel, and minimum diameters of 0.7µm and maximum diameters of 1.5µm (Image 7) in the soluble collagen.

In gelatin gel, on the other hand, no fibers were detected.

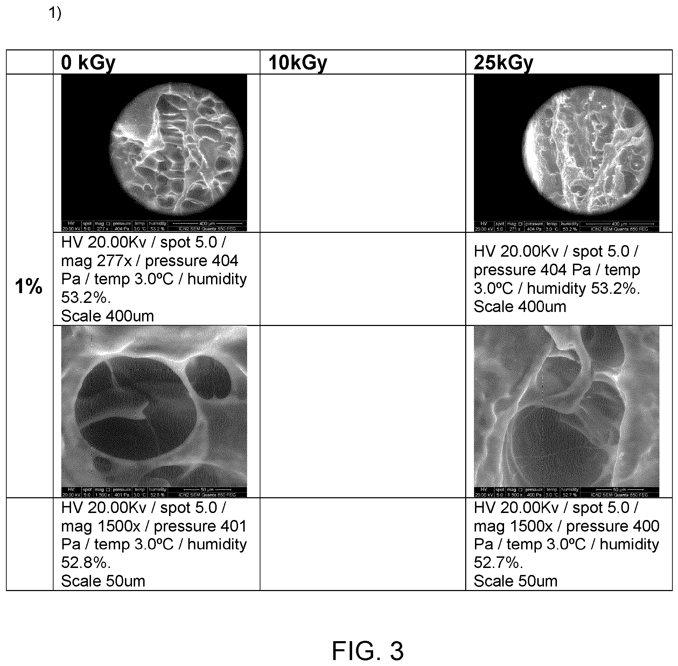

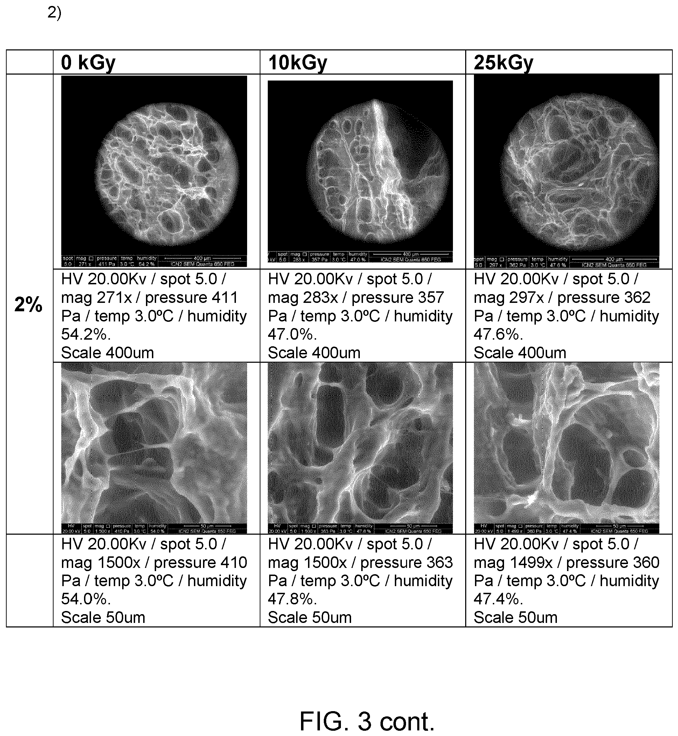

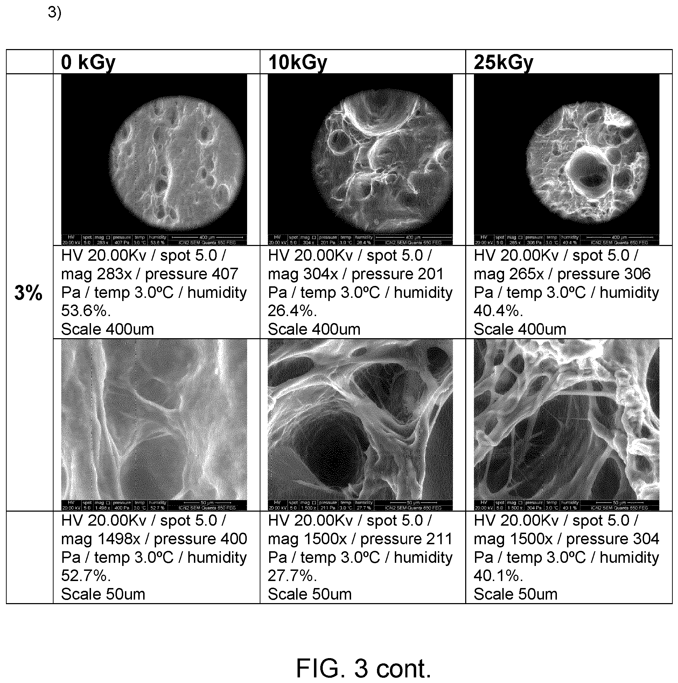

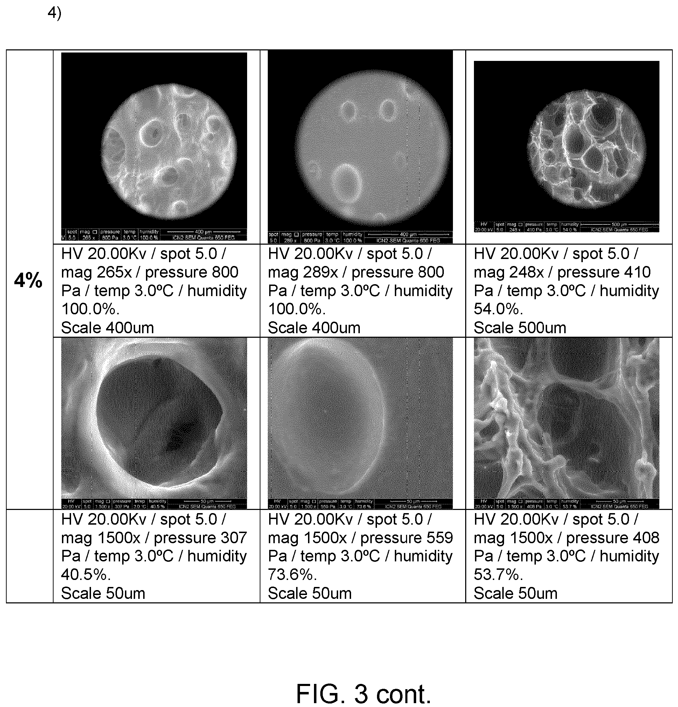

Subsequently, an attempt was made to resolubilize the acidic medium gel with deionized water adjusted to pH 2.5 with 1M HCl, leaving it for 24 hours in agitation. It was diluted to a final concentration of 0.05%, placed on the slide, allowed to dry and stained with 0.1% Sirius Red stain for 1 hour. In the case of the hydrogel, agglomerates of fibers were observed (FiG.3, Image 8) with diameters from 10µm to more than 300µm. Of these, some fibers could be observed unwinding from the agglomerate (FIG. 2. Image 9). In soluble collagen, on the other hand, no fibers were observed.

### Example 4B internal structure of hydrogels containing 1, 2, 3 and 4% collagen by means of ESEM technology.

To study the internal structure of the hydrogels, environmental scanning electron microscopy (ESEM) technology was used, which allows the temperature of the samples to be varied and, combined with the water vapor pressure introduced into the equipment, the humidity of the samples can be controlled (from 1% to 100% relative humidity). This allows to know the structure of the samples as close as possible to reality, without modifying them.

The samples were cut crosswise and placed vertically on the SEM stand. They were observed both at 100% humidity and at lower humidities (between 30% and 60%), thus allowing them to dry, and the pores they contain to be observed with greater contrast.

Porosity is important to facilitate cell internalization. FIG. 3 is an ESEM photographs at two different scales of the hydrogels. The photographs in FIG. 3 show how, as the percentage of collagen increases, the porosity of the structure decreases, but it still has large pores (greater than 50 µm) and interconnection can be seen in many of them.

The photographs shown correspond to images taken at a humidity of approximately 50%, and for each type of hydrogel are included at two different scales, which show the fibrillar structure of the collagen and the connection of these fibers.

### Example 5: Rheology of hydrogels of the present invention

### Example 5A: Rheology of hydrogels combining different collagen concentrations and irradiation doses.

Hydrogels were prepared from masses with collagen percentages from 1% to 10% following Example 2, which were subsequently analyzed without irradiation, irradiated by beta rays at an intensity of 10kGy and at 25kGy to see the effect of these variables on the rheology of the hydrogels.

Protocol for the analysis: For this purpose, a Modulate Advanced Rheometer System MARS 40 (Thermo Haake) equipped with a 20mm plate-plate rotor was used. An oscillatory stress sweep test was performed in a stress range (τ) 1 Pa to 15000Pa at a fixed frequency of 1Hz maintaining a distance between plates of 50% of the height of the initial thickness of the sample of hydrogel (usually between 1.5 and 2.5 mm) in the nonirradiated samples and 30% in the irradiated samples, due to an increase in fracturability, at a measurement temperature of 15.0°C. Prior to the test, the sample was left to temper for 10 minutes between the plates once the set gap had been reached, in order to ensure the measurement temperature.

The stress sweep at a fixed frequency makes it possible to identify the linear viscoelasticity zone.

Oscillatory measurements in the linear viscoelasticity zone are of great importance because the response of the material is only dependent on the material structure and independent of rheological parameters such as stress or strain.

During the test, the value of G' and G" (ordinate axis) is plotted as a function of stress (abscissa axis).

The elastic or storage modulus (G') is associated with the energy stored in the material while the viscous or loss modulus (G") is associated with the energy dissipated by the material.

**Table 4 Rheology data of the hydrogels of the invention after performing a stress sweep at a frequency of 1 Hz.**

| Collagen concentration | Irradiation dose | G' (Pa)* | G" (Pa)* |
|---|---|---|---|
| 1% | 0 kGy | 860 | 40 |
| 1% | 25 kGy | 2770 | 90 |
| 2% | 0 kGy | 2200 | 120 |
| 2% | 10 kGy | 8880 | 190 |
| 2% | 25 kGy | 6300 | 180 |
| 3% | 0 kGy | 5640 | 245 |
| 3% | 10 kGy | 17820 | 360 |
| 3% | 25 kGy | 25640 | 1210 |
| 4% | 0 kGy | 6840 | 350 |
| 4% | 10 kGy | 31290 | 900 |
| 4% | 25 kGy | 38480 | 1735 |
| 7,50% | 0 kGy | 13330 | 940 |
| 7,50% | 25 kGy | 41990 | 2265 |
| 10% | 0 kGy | 20980 | 2065 |
| 10% | 25 kGy | 66340 | 4730 |

| | | | |
|---|---|---|---|
| *Corresponds to the average value of G' and G" during the first 100 seconds of analysis, ensuring that it is in the linear phase of the curve. | | | |

The elastic modulus of hydrogels increases as the collagen dose increases, ranging from 860 Pa in a 1% gel to 20980 Pa in a 10% collagen content gel.

The same happens when the gel is irradiated, as the irradiation dose increases, the material crosslinks and the elastic modulus increases significantly.

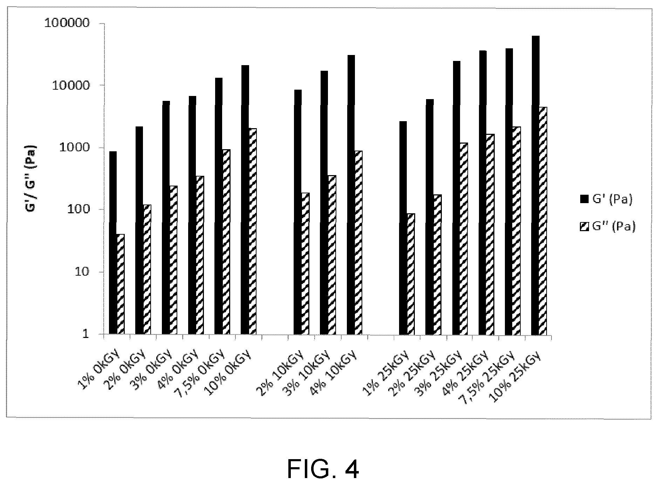

FIG. 4 also shows these rheology data of the hydrogels of the invention.

In this way, a range from 860 Pa corresponding to the 1% gel without irradiation to 66340 Pa corresponding to the 10% gel irradiated at 25 kGy is achieved; this allows to customize the elastic modulus by varying these parameters.

### Example 5B: comparative rheology of 4% hydrogel vs. 4% gelatin and soluble collagen gel

For rheological analysis hydrogels were prepared from a 4% collagen mass and irradiated at 25kGy following example 2, and from gelatin and soluble collagen gels were prepared in the same way as in example 3 part 2 and subsequently irradiated at 25kGy. The rheology measurement followed the same protocol as in example 4 part 1 (rheology of hydrogels).

It is important to maintain the firmness of the gels for use in biomedical applications to be used as a support for cell growth. This requires a high elastic modulus (G').

The results of the stress sweep test are presented in Table 5.

**Table 5:**

| | Concentration collagen / gelatin | Dose Irradiation | G' (Pa) | G" (Pa) |
|---|---|---|---|---|
| Hydrogel | 4% | 25 kGy | 38480 | 1735 |
| Gelatin | 4% | 25 kGy | 13815 | 190 |
| Soluble collagen | 0,5% | 25 kGy | 54 | 7 |

The elastic modulus of the hydrogels was significantly higher than the other two gels tested. In the case of soluble collagen, it is not possible to achieve as high a collagen concentration as with the other materials, but the G' value of the irradiated product was much lower.

### Example 6. Part 1. Cell culture with fibroblastic line

Hydrogels were obtained from a mass with 4% collagen irradiated at 25 KGy as mentioned in Example 2, after which in vitro assays were performed. For this purpose, gels were equilibrated in enriched Dulbecco's Modified Eagle Medium (DMEM) for 24 hours. Fibroblasts from murine subcutaneous connective tissue (I929 line) were cultured, resuspending a total of 100,000 cells/cm².

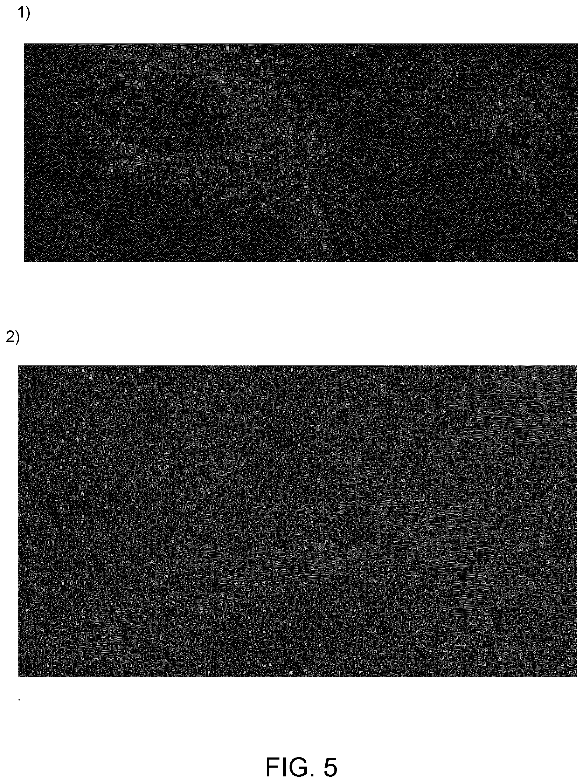

For correct visualization of the cells, a DiIC₁₂ staining protocol (membrane labeling) was performed, followed by visualization using an inverted fluorescence microscope (TxRed filter). FIG. 5 Images 1 and 2 show the cell culture on collagen gels after DiIC12 staining with the I929 line obtained 2 and 5 days after seeding, respectively.

### Example 6. Part 2. Cell culture with human renal embryonic line

In turn, in vitro experiments were performed on collagen hydrogels of different collagen concentration, irradiation dose and elastic modulus (see Table 6); whose obtention process is mentioned in Examples 2 and 4A. Once the hydrogels were obtained, they were equilibrated in enriched Dulbecco's Modified Eagle Medium (DMEM) for 24 hours and human renal embryonic cells (Line 293T) were cultured, with a confluence of 500,000 cells/cm².

**Table 6. Summary of collagen hydrogels tested in vitro with the 293T cell line.**

| Collagen concentration | Irradiation Dose | G' (Pa) |
|---|---|---|
| 2% | 0 kGy | 2200 |
| 2% | 10 kGy | 8880 |
| 2% | 25 kGy | 6300 |
| 4% | 0 kGy | 6840 |
| 4% | 10 kGy | 31290 |
| 4% | 25 kGy | 38480 |

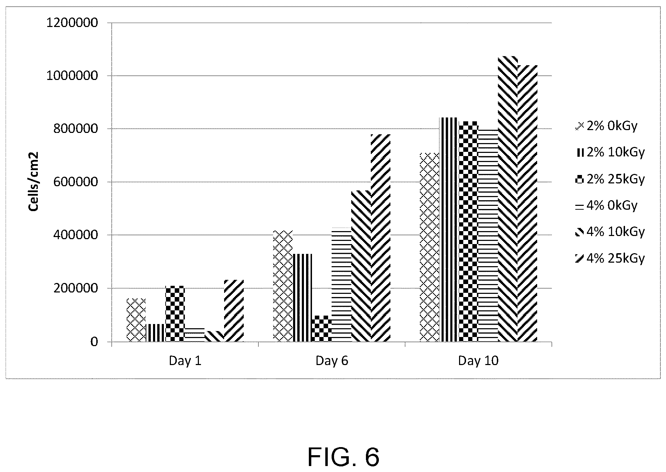

After culture in the different hydrogels, they were decellularized by a combined treatment with trypsin and collagenase (first incubation of 20 minutes with Trypsin-EDTA 0.05%; followed by a second incubation with collagenase 500U/ml) until total dissolution of the gel. After this, cell counting was performed by staining with TrypanBlue and counting with a Neubauer chamber. FIG. 6 shows the cell counts obtained after 1, 6 and 10 days of growth on the different gels. The gels with a higher elastic modulus (G') (those with 4% collagen treated at 10 and 25 kGy), present both, at 6 and 10 days, a higher cell confluence.

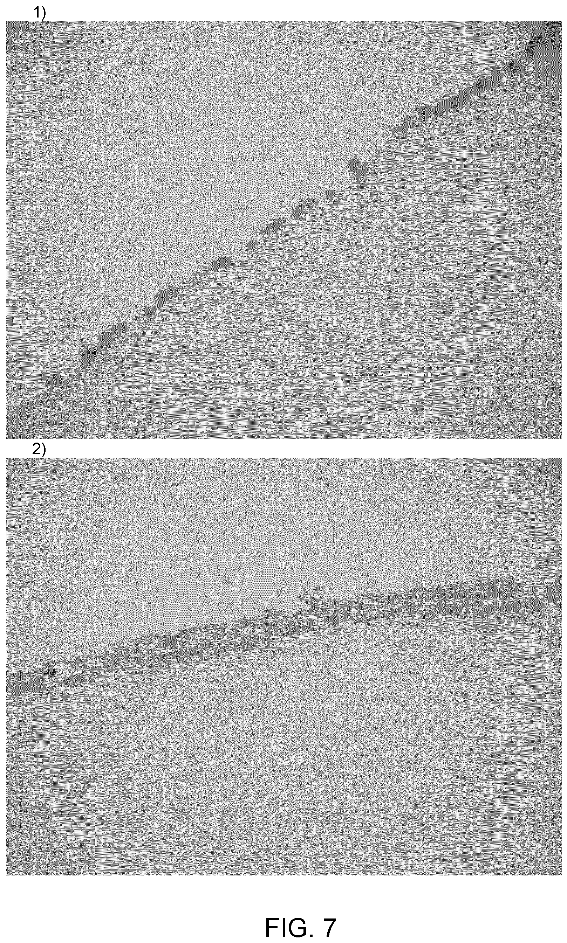

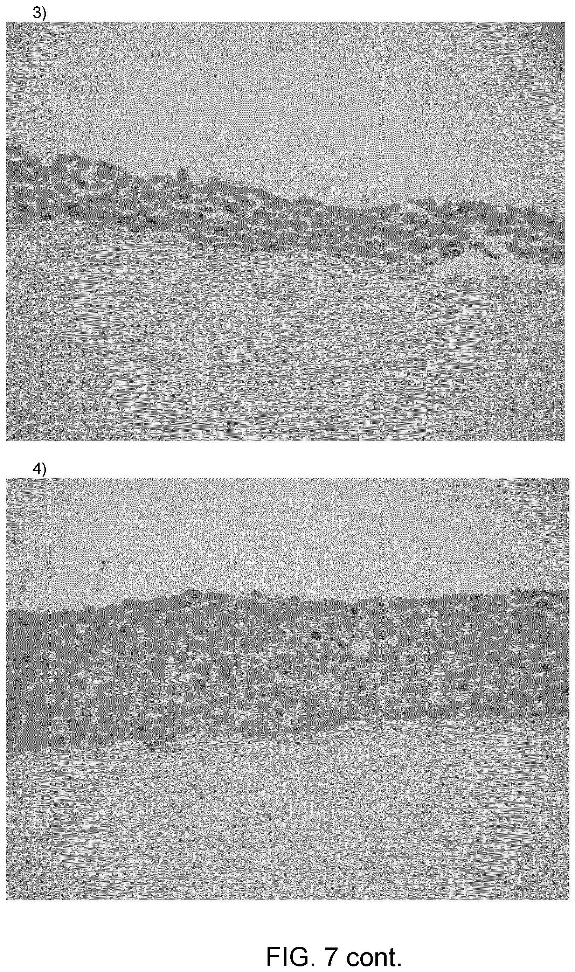

FIG. 7 shows the 293T cell line on 4% hydrogels previously irradiated at 25 KGy. Cells were stained by hematoxylin-eosin. At day 1 (Fig 8.1) cells were adhered to the hydrogel forming a monolayer. At both day 6 (Fig 8.2) and 10 (Fig 8.3) some areas covered by a multilayer with 2-3 layers of cells were observed. At day 10 (Fig 8.4) some zones with multiple layers (14-15 layers) of cells were detected.

### Example 7: Stability in culture medium

Hydrogels were prepared following example 2 from a 2% mass and irradiated at 10kGy and 25kGy to evaluate stability in culture medium. For this purpose, they were immersed in DMEM enriched culture medium and incubated in an oven at 37°C for 7 days. After the incubation period, the diameter of the hydrogel was slightly reduced, but the consistency did not substantially change.

FIG. 8 image 1 shows the hydrogels before being immersed in culture medium, and image 2 after 7 days of incubation, where the reduction in the diameter of the hydrogels can be seen.

FIG. 8, images 3, 4 and 5 show the hydrogel made from 2% mass of collagen irradiated at 10kGy before immersion in culture medium, the hydrogel from 2% mass of collagen irradiated at 10kGy after 7 days of incubation and the hydrogel from 2% mass of collagen irradiated at 25kGy after 7 days of incubation respectively, where it can be seen that the firmness and structure is not substantially affected.

In hydrogels prepared from a higher mass concentration of collagen the reduction in diameter is lower, this is shown in FIG. 8 images 6 and 7, corresponding to hydrogels prepared from a 4% mass of collagen irradiated at 10kGy and 25kGy before immersion in medium (image 6) and after 7 days of incubation (image 7).

### Citation List

### Patent Literature

- US56706684B1

### Non Patent Literature

- P.N. Oliveira et al; "Self-crosslinked fibrous collagen/chitosan blends: Processing, properties evaluation and monitoring of degradation by bi-fluorescence imaging"; International Journal of Biological Macromolecules, 2019, pp. 353-367.

## Claims

1. A collagen hydrogel which comprises a 3D fibrillar structure with micropores obtainable by a process comprising the following steps:
a) providing an acidic mass of native Type I collagen fibers dispersed in water in a concentration comprised between 1-15% in weight;
b) neutralizing the acidic mass to adjust the pH to physiological pH; and
c) applying beta radiation to the neutralized mass of step b) in a range of up to 50 KGy;
wherein:
- the acidic mas has a pH comprised between 0.5-5;
- the acidic mass comprises fibers, wherein at least 90% of the total mass of the fibers have a length comprised in a range from 10 µm to 2500 µm and a fiber diameter comprised in a range from 5 to 80 µm measured at a pH between 1 and 2; and
wherein the median of the fiber length in the total mass of fibers of collagen is comprised between 100 and 900 µm.

2. The collagen hydrogel according to claim 1, wherein at least 80 % of the mass of the fibers in the acidic mass have a fiber length comprised in a range from 50 to 1300 µm.

3. The collagen hydrogel according to claim 2, wherein the median of the fiber length in the total mass of fibers of collagen is comprised between 100 and 500 µm.

4. The collagen hydrogel according to claim 3, wherein the median of the fiber length is the total mass of fibers of collagen is comprised between 200 and 400 µm.

5. The collagen hydrogel according to any of the claims 1-4, wherein at least 80% of the mass of the fibers in the acidic mass have a fiber diameter comprised in a range from 5 to 50 µm.

6. The collagen hydrogel according to any of the claims 1-5, wherein the median of the fiber diameter in the total mass of fibers of collagen is comprised in range from 10 to 25 µm.

7. The collagen hydrogel according to any of the claims 1-6, wherein the acidic mass has a concentration comprised between 1-10 % in weight of native Type I collagen.

8. The collagen hydrogel according to claim 7, wherein the acidic mass has a concentration comprised between 2-4% in weight of native Type I collagen.

9. The collagen hydrogel according to any of the claims 1-8, wherein the acidic mass has a pH comprised between 1 and 3.

10. The collagen hydrogel according to any of the claims 1-9, wherein the radiation is comprised between 10 and 25 KGy.

11. A process for preparing a collagen hydrogel as defined in any of the claims 1-10, which comprises the following steps:
a) providing an acidic mass of native Type I collagen fibers dispersed in water as defined in any of the claims 1-10;
b) neutralizing the acidic mass to adjust the pH to physiological pH; and
c) applying beta radiation to the neutralized mass of step b) in a range of up to 50 KGy.

12. The process according to claim 11, further comprising preparing the acidic mas by a process which comprises the following steps:
a) washing and cutting up a tissue containing collagen;
b) chemically macerating the chopped tissue in a controlled manner;
c) washing the product obtained from step b) with water;
d) adjusting the pH of the washed product obtained in c) to values between 0.5 and 5 to swell the washed product of step c);
e) mechanically minced the product of step d); and
f) dispersing in water to a concentration preferably between 1% and 15%.

13. Use of the collagen hydrogel according to any of the claims 1-10, as support for cell growth or for as scaffold for cultured meat.

14. A method for the cultivation of biological material, comprising the following steps:
a) Providing a carrier material which is a collagen hydrogel material according to any of the claims 1-10;
b) Contacting said biological material with the collagen hydrogel material of step a)
and
c) Incubating said biological material with the collagen hydrogel material under cultivation conditions.

15. Collagen hydrogel as defined in any of the claims 1-10, comprising adhered cell layers for use in biomedical applications.

## Patentansprüche

1. Ein Kollagenhydrogel, welches eine 3D-Fibrillenstruktur mit Mikroporen umfasst, die durch ein Verfahren erhalten werden kann, das die folgenden Schritte umfasst:
a) Bereitstellen einer sauren Masse aus Fasern von nativem Typ-I-Kollagen, die in Wasser in einer Konzentration zwischen 1-15 Gew.- % dispergiert sind;
b) Neutralisieren der sauren Masse, um den pH-Wert auf einen physiologischen pH-Wert einzustellen; und
c) Aufbringen von Beta-Strahlung auf die neutralisierte Masse von Schritt b) in einem Bereich von bis zu 50 KGy;
wobei:
- die saure Masse einen pH-Wert von zwischen 0,5 und 5 hat;
- die saure Masse Fasern umfasst, wobei mindestens 90 % der gesamte Masse der Fasern eine Länge in einem Bereich von 10 µm bis 2500 µm und einen Faserdurchmesser in einem Bereich von 5 bis 80 µm, gemessen bei einem pH-Wert zwischen 1 und 2, haben; und
wobei der Median der Faserlänge in der gesamten Masse der Kollagenfasern zwischen 100 und 900 µm liegt.

2. Das Kollagenhydrogel nach Anspruch 1, wobei mindestens 80 % der Masse der Fasern in der sauren Masse eine Faserlänge in einem Bereich von 50 bis 1300 µm haben.

3. Das Kollagenhydrogel nach Anspruch 2, wobei der Median der Faserlänge in der gesamten Masse der Kollagenfasern zwischen 100 und 500 µm liegt.

4. Das Kollagenhydrogel nach Anspruch 3, wobei der Median der Faserlänge in der gesamten Masse der Kollagenfasern zwischen 200 und 400 µm liegt.

5. Das Kollagenhydrogel nach einem der Ansprüche 1 bis 4, wobei mindestens 80 % der Masse der Fasern in der sauren Masse einen Faserdurchmesser in einem Bereich von 5 bis 50 µm haben.

6. Das Kollagenhydrogel nach einem der Ansprüche 1 bis 5, wobei der Median des Faserdurchmessers in der gesamten Masse der Kollagenfasern im Bereich von 10 bis 25 µm liegt.

7. Das Kollagenhydrogel nach einem der Ansprüche 1 bis 6, wobei die saure Masse eine Konzentration zwischen 1 und 10 Gew.- % an nativem Typ-I-Kollagen hat.

8. Das Kollagenhydrogel nach Anspruch 7, wobei die saure Masse eine Konzentration zwischen 2 und 4 Gew.- % an nativem Typ-I-Kollagen hat.

9. Das Kollagenhydrogel nach einem der Ansprüche 1 bis 8, wobei die saure Masse einen pH-Wert zwischen 1 und 3 hat.

10. Das Kollagenhydrogel nach einem der Ansprüche 1 bis 9, wobei die Strahlung zwischen 10 und 25 KGy liegt.

11. Ein Verfahren zur Herstellung eines Kollagenhydrogels wie in einem der Ansprüche 1 bis 10 definiert, das die folgenden Schritte umfasst:
a) bereitstellen einer sauren Masse aus Fasern von nativem Typ-I-Kollagen, die in Wasser dispergiert sind, wie in einem der Ansprüche 1 bis 10 definiert;
b) neutralisieren der sauren Masse, um den pH-Wert auf einen physiologischen pH-Wert einzustellen; und
c) anwenden von Beta-Strahlung auf die neutralisierte Masse von Schritt b) in einem Bereich von bis zu 50 KGy.

12. Das Verfahren nach Anspruch 11, ferner umfassend die Herstellung der sauren Masse durch ein Verfahren, das die folgenden Schritte umfasst:
a) Waschen und Schneiden eines kollagenhaltigen Gewebes;
b) chemiches Aufweichen des geschnittenen Gewebes auf kontrollierte Weise;
c) Waschen des aus dem Schritt b) erhaltenen Produkts mit Wasser;
d) Einstellen des pH-Werts des in c) erhaltenen gewaschenen Produkts auf Werte zwischen 0,5 und 5, um das gewaschene Produkt von Schritt c) zu quellen;
e) das Produkt von Schritt d) wird mechanisch zerkleinert; und
f) Dispergieren in Wasser auf eine Konzentration vorzugsweise zwischen 1 % und 15 %.

13. Verwendung des Kollagenhydrogels nach einem der Ansprüche 1 bis 10 als Träger für das Zellwachstum oder als Gerüst für kultiviertes Fleisch.

14. Ein Verfahren zum Kultivieren von biologischem Material, umfassend die folgenden Schritte:
a) Bereitstellen eines Trägermaterials, welches ein Kollagenhydrogelmaterial nach einem der Ansprüche 1 bis 10 ist;
b) Kontaktieren des biologischen Materials mit dem Kollagenhydrogelmaterial von Schritt a) und
c) Inkubation des biologischen Materials mit dem Kollagenhydrogelmaterial unter Kultivierungsbedingungen.

15. Kollagenhydrogel wie in einem der Ansprüche 1 bis 10 definiert, umfassend haftende Zellschichten zur Verwendung in biomedizinischen Anwendungen.

## Revendications

1. Un hydrogel de collagène qui comprend une structure fibrillaire 3D avec des micropores pouvant être obtenue par un procédé comprenant les étapes suivantes :
a) fournir une masse acide de fibres de collagène de type I natif dispersées dans l'eau à une concentration comprise entre 1 et 15 % en poids ;
b) neutraliser la masse acide pour ajuster le pH au pH physiologique ; et
c) appliquer du rayonnement bêta à la masse neutralisée de l'étape b) dans une plage allant jusqu'à 50 KGy ;
dans lequel :
- la masse acide a un pH compris entre 0,5 et 5 ;
- la masse acide comprend des fibres, dans lequel au moins 90 % de la masse totale des fibres ont une longueur comprise dans une plage de 10 µm à 2500 µm et un diamètre de fibre compris dans une plage de 5 à 80 µm mesuré à un pH entre 1 et 2 ; et
dans lequel la médiane de la longueur de fibre dans la masse totale de fibres de collagène est comprise entre 100 et 900 µm.

2. L'hydrogel de collagène selon la revendication 1, dans lequel au moins 80 % de la masse des fibres dans la masse acide ont une longueur de fibre comprise dans une plage de 50 à 1300 µm.

3. L'hydrogel de collagène selon la revendication 2, dans lequel la médiane de la longueur de fibre dans la masse totale de fibres de collagène est comprise entre 100 et 500 µm.

4. L'hydrogel de collagène selon la revendication 3, dans lequel la médiane de la longueur de fibre dans la masse totale de fibres de collagène est comprise entre 200 et 400 µm.

5. L'hydrogel de collagène selon l'une quelconque des revendications 1 à 4, dans lequel au moins 80 % de la masse des fibres dans la masse acide ont un diamètre de fibre compris dans une plage de 5 à 50 µm.

6. L'hydrogel de collagène selon l'une quelconque des revendications 1 à 5, dans lequel la médiane du diamètre de fibre dans la masse totale de fibres de collagène est comprise dans la plage de 10 à 25 µm.

7. L'hydrogel de collagène selon l'une quelconque des revendications 1 à 6, dans lequel la masse acide a une concentration comprise entre 1 et 10 % en poids de collagène de type I natif.

8. L'hydrogel de collagène selon la revendication 7, dans lequel la masse acide a une concentration comprise entre 2 et 4 % en poids de collagène de type I natif.

9. L'hydrogel de collagène selon l'une quelconque des revendications 1 à 8, dans lequel la masse acide a un pH compris entre 1 et 3.

10. L'hydrogel de collagène selon l'une quelconque des revendications 1 à 9, dans lequel le rayonnement est compris entre 10 et 25 KGy.

11. Un procédé de préparation d'un hydrogel de collagène tel que défini dans l'une quelconque des revendications 1 à 10, qui comprend les étapes suivantes :
a) fournir une masse acide de fibres de collagène de type I natif dispersées dans l'eau telle que définie dans l'une quelconque des revendications 1 à 10 ;
b) neutraliser la masse acide pour ajuster le pH au pH physiologique ; et
c) appliquer du rayonnement bêta à la masse neutralisée de l'étape b) dans une plage allant jusqu'à 50 KGy.

12. Le procédé selon la revendication 11, comprenant en outre la préparation de la masse acide par un procédé qui comprend les étapes suivantes :
a) laver et découper un tissu contenant du collagène ;
b) macérer chimiquement le tissu coupé en morceaux de manière contrôlée ;
c) laver le produit obtenu à l'étape b) avec de l'eau ;
d) ajuster le pH du produit lavé obtenu en c) à des valeurs comprises entre 0,5 et 5 pour gonfler le produit lavé de l'étape c) ;
e) le produit de l'étape d) est mécaniquement haché ; et
f) disperser dans l'eau à une concentration comprise de préférence entre 1 % et 15 %.

13. Utilisation de l'hydrogel de collagène selon l'une quelconque des revendications 1 à 10, en tant que support pour la croissance cellulaire ou en tant qu'échafaudage pour de la viande cultivée.

14. Un procédé de culture de matériel biologique, comprenant les étapes suivantes :
a) Fournir un matériau de support qui est un matériau d'hydrogel de collagène selon l'une quelconque des revendications 1 à 10 ;
b) Mettre en contact ledit matériau biologique avec le matériau d'hydrogel de collagène de l'étape a) et
c) Incuber ledit matériau biologique avec le matériau d'hydrogel de collagène dans des conditions de culture.

15. Hydrogel de collagène tel que défini dans l'une quelconque des revendications 1 à 10, comprenant des couches cellulaires adhérentes pour une utilisation dans des applications biomédicales.
